**Europäisches Patentamt**

**European Patent Office**

**Office européen des brevets**

(11) Veröffentlichungsnummer: **0 061 993**

**A1**

(12) ## EUROPÄISCHE PATENTANMELDUNG

(21) Anmeldenummer: **82730043.5**

(22) Anmeldetag: **01.04.82**

(51) Int. Cl.³: **A 61 F 1/03**

(30) Priorität: **01.04.81 DE 3113531**
**23.10.81 DE 3142730**

(43) Veröffentlichungstag der Anmeldung:
**06.10.82 Patentblatt 82/40**

(84) Benannte Vertragsstaaten:
**IT**

(71) Anmelder: **MECRON MEDIZINISCHE PRODUKTE GMBH**
**Nunsdorfer Ring 25-27**
**D-1000 Berlin 48(DE)**

(72) Erfinder: **Kranz, Curt, Dipl.-Ing.**
**Landshuter Strasse 5**
**D-1000 Berlin 30(DE)**

(74) Vertreter: **Christiansen, Henning, Dipl.-Ing.**
**Unter den Eichen 108a**
**D-1000 Berlin 45(DE)**

(54) **Gelenkprothese.**

(57) Ein von einem Anpaßteil (1) gehaltenes Prepreg-Teil (2) mit aushärtbaren Bereichen vorgesehenen, Gelenk-prothese, in den Knochenhohlraum passen und mit Hilfe von für ein Strömungsmittel undurchlässigen und aufblähbaren Anpreßmittel (4) gegen die Innenwandung eines Knochens preßbar und in dieser fixierten Position aushärtbar sind.

FIG.1

EP 0 061 993 A1

MECRON                                    30. März 1982
Medizinische Produkte GmbH

D-1000 Berlin

ME31.1-EU

---

Gelenkprothese

---

B e s c h r e i b u n g

Die Erfindung betrifft eine in der orthopädischen Chirurgie häufig angewendete Endoprothese (Gelenkprothese). Sie
ist für alle Arten von Endoprothesen anwendbar, obgleich
sich die nachfolgende Beschreibung eines Ausführungsbeispiels ausschließlich auf eine Hüftgelenkprothese bezieht.

Die verschiedensten Indikationen können es angebracht erscheinen lassen, einem Patienten einen Gelenkersatz zu implantieren. Die herkömmlichen Endoprothesen haben aber nur eine Lebensdauer von etwa fünf bis acht Jahren. Die häufigste Versagensform dieser Prothesen ist die Lockerung aus dem Zementköcher bzw. bei zementlos eingesetzten Prothesen aus der Kortikalis des Femur. Die herkömmliche zementlos eingesetzte Endoprothese hat den Nachteil, daß der in den Knochen (z.B. Femur) hineinragende Schaft durch die notwendige Konfektionierung in einer bestimmten Anzahl von Größen und durch das Fehlen einer weiteren Krümmung aus der einzigen Krümmungsebene heraus (keine Unterscheidung von rechts und links) ungenügend an die Form des Markhohlraums angepaßt ist. Die daraus resultierenden punktuellen Auflagen führen zu örtlich sehr hohen Querbelastungen sowie Entlastungen der Kortikalis in Längsrichtung und damit zu Resorptionen im oberen Bereich des Prothesenansatzes. Die zementierte Endoprothese ist zwar durch den Zement formschlüssig in dem Hohlraum verankert, jedoch stellt der Verbund zwischen Prothesenschaft und Zementköcher ein um etwa eine Zehnerpotenz biegesteiferes Bauteil dar als die Kortikalisröhre des Knochens. Die Folge ist eine Umorientierung des Kraftflusses in der Kortikalis mit einer großflächigen Entlastung sowie örtlichen Überbelastungen quer zur Faserorientierung.

Dementsprechend liegt der Erfindung die Aufgabe zugrunde, eine Gelenkprothese zu schaffen, die in Form und Steifigkeitsverteilung ohne wesentliche Kompromisse an die Kortikalis des Knochens individuell angepaßt werden kann und in der Lage ist, die für den allgemeinen Bewegungsablauf erforderlichen Kräfte sowohl statisch als auch dynamisch zu ertragen und auf physiologische Weise in die Kortikalis einzuleiten, so daß Lockerungen in geringerem Maße zu erwarten sind als bei herkömmlichen Prothesen.

Diese Aufgabe wird gelöst durch die Gelenkprothese mit den Merkmalen des Patentanspruches 1. Vorteilhafte Weiterbildungen sind in den Unteransprüchen angegeben. Der dabei verwendete Begriff "Prepreg" ist die übliche Bezeichnung für bereits mit einem Matrix-Kunststoff benetzte Faser-Gelege (Glas, Kohle, Aramid od. ähnliches), die noch nicht ausgehärtet sind, d.h. deren Polymerisationsprozeß erst durch äußere Einflüsse wie Wärme, ultraviolettes Licht, Licht, Ultraschall oder ähnliches angeregt werden muß. Der dabei verwendete Matrix-Kunststoff ist ein Kunststoff, der in flüssiger Form mit Fasern oder Geweben in Verbindung gebracht wird und nach der Aushärtung' einen Verbund zwischen den einzelnen Fibern bzw. Fasern herstellt. Diese sind in dem Gelege aufeinandergeschichtet oder in Gestalt gerichteter Gewebe mit dem Ziel enthalten, genau definierte, gerichtete Steifigkeiten sowie Festigkeiten des in Verbindung mit geeigneten Matrix-Kunststoffen sich ergebenden Verbundes zu erzeugen.

Bevorzugt besteht das Prepreg-Teil aus besonders zugeschnittenen endseitig befestigten Streifen, welche die tragende und krafteinleitende Funktion der Prothese übernehmen und erst während der Operationsphase durch die Verwendung von ultraviolettem Licht oder Ultraschall ausgehärtet werden und damit exakt an die Kortikalis angepaßt werden. Die Kraftüberleitung auf einen Gelenkkopf, beispielsweise eine Aluminiumoxid-Keramikkugel geschieht durch ein besonders geformtes Anpaßteil (Adapter), das bevorzugt aus einer körperverträglichen Titanlegierung besteht. Durch die Anwendung eines bereits in der Zahnmedizin gebräuchlichen Kunststoffs, der durch ultraviolettes Licht aushärtet, zum Einbetten der Fasern der Prepreg-Streifen entstehen keine zusätzlichen Komplikationen im Zusammenhang mit der Körperverträglichkeit des gesamten Implantats. Vielmehr ist gleiche oder bessere Verträglichkeit mit dem menschlichen Körper zu erwarten als bei herkömmlichen Prothesen. Alle für die Implantation notwendigen Hilfsstoffe und Vorrichtungen können aus gebräuchlichen sterilisierbaren Stoffen gefertigt werden. Als Faserwerkstoff wird Glas, Aramid oder bei Aushärtung durch Ultraschall auch Carbon verwendet.

Die größere Lebensdauer durch verminderte Gefahr der Lockerung und der damit verbundenen Bruchgefahr bei herkömmlichen Prothesen verdankt die erfindungsgemäße Gelenkprothese der besseren individuellen Anpaßbarkeit der tragenden und krafteinleitenden Struktur des Implantats (Prepreg-Teil) an die physiologischen Gegebenheiten (Form, Rauigkeit) des Markhohlraums während des Einsetzens der Gelenkprothese im Verlauf einer Operation sowie der an die physiologischen Bedingungen (Biegesteifigkeit) besser angepaßten Krafteinleitung in die Kortikalis des Knochens bei weitgehender Vermeidung von unphysiologischen Kräften senkrecht zur Faserorientierung der Kortikalis. Dabei ist die neue Gelenkprothese ohne wesentlich größeren Aufwand zu implantieren als die herkömmlichen Prothesen, und sie ist im Falle einer eventuellen notwendigen Reoperation ohne großen Aufwand zu entfernen. Sie ist in allen Fällen einsetzbar, in denen derzeit die konventionelle Form der Prothese benutzt wird, und im besonderen, wenn eine physiologische Abnormität die Einpassung einer konventionellen Prothese erschwert oder unmöglich macht, weil diese nur in bestimmten Abmessungen konfektioniert auf dem Markt angeboten wird. Durch die hohe Anpassungsfähigkeit ist das Anwendungsspektrum bedeutend weiter als das der herkömmlichen Prothesenform.

Anhand der Zeichnungen wird ein bevorzugtes Ausführungsbeispiel der Erfindung beschrieben, bei welchem das Prepreg-Teil und die Anpreßmittel durch voneinander getrennte Teile gebildet sind, während die Einführhilfe und das Einführungsteil für das Aushärtemittel durch ein gemeinsames, beide Funktionen erfüllendes Teil gebildet sind, das hohl ist und durchlässig, sowohl für ein Strömungsmittel zum Aufblähen der Anpreßmittel als auch für Strahlung von einem zum Aushärten des Prepreg-Teiles vorgesehenen Strahler, der in dem hohlen Teil, das zugleich Einführhilfe und Einführungsteil ist, geführt ist. Es ist ersichtlich, daß die Art der Zuordnung verschiedener Funktionen zu unterschiedlichen bzw. gemeinsamen Teilen, wie es im folgenden beschrieben und dargestellt ist, nur eine der möglichen Ausführungsformen wiedergibt. Es zeigen:

Figur 1 im Längsschnitt dargestellt eine Gelenkprothese als Ausführungsbeispiel der Erfindung,

Figur 2 die Hauptbestandteile derselben Gelenkprothese, die in eine Kortikalis eingesetzt, dort aber noch nicht befestigt ist,

/6

Figur 3 die Anordnung nach Figur 2, jedoch während des Aushärtens, und

Figur 4 die fertig eingesetzte und vervollständigte Gelenkprothese.

In den untereinander mit identischen Bezugzeichen versehenen Figuren ist übereinstimmend die folgende Anordnung vorhanden:

Ein zweiteiliges Anpaßteil 1, welches eine Klemmverbindung mit einem Prepreg-Teil 2 bildet, ist mit einem Halteteil 1a versehen, auf dessen Gewindehals 1b ein Überwurfteil 1c aufgeschraubt ist. Das Prepreg-Teil 2 ist mit seinem oberen Bereich zwischen den Teilen 1a und 1c eingespannt. Eine durch den Gewindehals des Anpaßteils 1 hindurchgeführte, in das Prepreg-Teil hineinragende, Einführhilfe 3 stellt zugleich das sogenannte Einführungsteil dar, auf das verschiedentlich getrennt Bezug genommen wird. Anpreßmittel 4 sind im Innerern des Prepreg-Teils in Gestalt eines Folienbeutels und eines am unteren Ende des Einführungsteiles 3 zusammen mit diesem verschlossenen, aufblähbaren Schlauches vorgesehen. Ein in das Einführungsteil hineinführbarer UV-Licht- oder Ultraschall-Strahler 5 ist mit Transportmitteln 6 versehen. Weiterhin sind ein Rohr 7, Dichtungen 8 und 9 und eine Montagehilfe 10 mit einem Stutzen 10a vorgesehen. Die Montagehilfe 10 besteht aus einem aufschraubbaren Adapter, welcher die notwendigen Anschlüsse zum Durchlaß von Strömungs- und Aushärtemitteln aufweist. Durch die Montagehilfe ist wegen der vergrößerten zugänglichen Oberfläche die Handhabung der Prothese bei der Implantation erleichtert.

/7

Das Prepreg-Teil 2 besteht aus Längsstreifen und/oder gegenläufigen spiraligen Streifen, die auf geeignete Weise verwoben sind, die zwischen dem Halteteil 1a und dem aufgeschraubten Überwurfteil 1c eingeklemmt sind und auf diese Weise den Eindruck einer gefiederten Glocke erwecken, die jedoch - falls die Aushärtung nicht auch hier erst in der Operationsphase erfolgt - in einem oberen Bereich, wo sie am Anpaßteil 1 befestigt ist, ausgehärtet ist, während sie in einem unteren Bereich 2b noch verformbar und unausgehärtet vorliegt.

In dem Gewindehals 1b steckt ein Rohr 7, in welches wiederum die Einführhilfe 3 so eingesteckt ist, daß das weitgehend für Strömungsmittel undurchlässige, insbesondere luftdichte und aufblähbare Anpreßmittel 4 an dessen oberen, dem Anpaßteil 1 zugewandten Ende eingeklemmt ist. Zur Aufblähung dieses Anpreßmittels 4, z.B. Folien- oder Gummisackes, kann durch den Stutzen 10a Luft durch die Montagehilfe 10, das Rohr 7, die Einführhilfe 3 und darin vorgesehene Löcher 3a in die Anpreßmittel 4 geleitet werden. Um das Entweichen dieser Luft an unerwünschten Stellen zu verhindern, sind die Dichtungen 8 und 9 vorgesehen. Zum Gewährleisten des Entweichens der Luft aus dem Hohlraum 11 ist eine nicht näher dargestellte Entlüftung in dem Anpaßteil 1 vorgesehen, durch welche auch Luft (zusätzlich oder anstelle der Druckluftanwendung über dem Stutzen 10a) abgesaugt werden kann. Die in die Anpreßmittel 4 gedrückte und/oder aus dem Hohlraum 11 gesaugte Luft ist das im vorliegenden Ausführungsbeispiel anzuwendende Strömungsmittel, durch welches die Anpreßmittel 4 aufblähbar sind, um die Streifen des Prepreg-Teiles 2 im Bereich

/8

2b gegen die Knocheninnenwand zu pressen. Um diesen angepaßten Zustand zu konservieren, ist ein Aushärtemittel
vorgesehen, nämlich der Strahler 5.

Zum Einführen ist das Einführungsteil 3 in Gestalt eines
Rohres aus Polyäthylen oder ähnlichem vorgesehen, das
zugleich als Einführhilfe für die noch flexiblen Teile 2
(im Bereich 2b) und 4 dient.

Als Transportmittel 6 für den Strahler 5 dient ein längliches, federnd nachgiebiges Element, mittels dessen dem
Strahler Energie zugeführt wird, entweder in Gestalt elektrischer Energie oder von ultraviolettem Licht, wenn das
Transportmittel 6 zugleich als Lichtleiter ausgebildet
ist. Der Transport des Strahlers 5 erfolgt mit definierter
Ausziehgeschwindigkeit durch nicht dargestellte Antriebsmittel, die an der Montagehilfe 10 befestigt sein können.
Die Prepreg-Streifen im Bereich 2b können so ausgebildet
sein, daß dazwischen Leerräume verbleiben, wenn sie an
eine Knocheninnenwand angedrückt sind. In diese Zwischenräume kann dann später Knochengewebe einwachsen. Als
Kunststoffmaterial der Prepregs kann z.B. ein Material
verwendet werden, welches unter der Bezeichnung "UVIOBOND"
in der zahnärztlichen Praxis Verwendung findet.

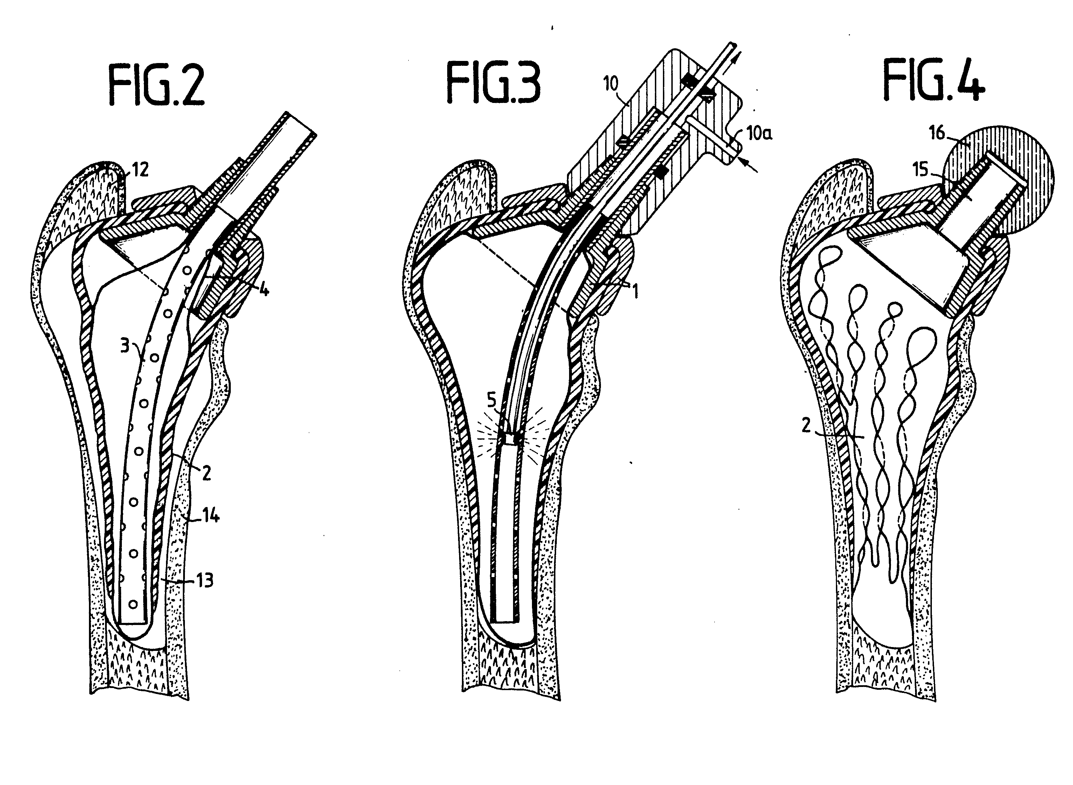

In Figur 2 ist gezeigt, wie die Gelenkprothese nach Figur
1 nach weitgehendem Ausräumen des Spongiosa 12 aus einem
Markhohlraum 13 einer Kortikalis 14 eingesetzt ist.

Figur 3 zeigt eine spätere Phase mit angepreßten Prepreg-
Streifen nach dem Eindrücken von Luft in den Stutzen 10a unter leichtem Überdruck von ungefähr 0,8 bar. Der UV-Licht- oder Ultraschall-Strahler 5 hat den Markhohlraum 13 schon zur Hälfte durchlaufen und durch seine die Teile 3 und 4 durchdringende Strahlung die Aushärtung eines Teils der Prepreg-Streifen 2 bewirkt. Dabei entsteht keine Wärme im Gegensatz zur Verwendung von Knochenzement. Die Aushärtung dauert ungefähr 6 bis 8 Minuten bei Verwendung von ultraviolettem Licht zur Aushärtung.

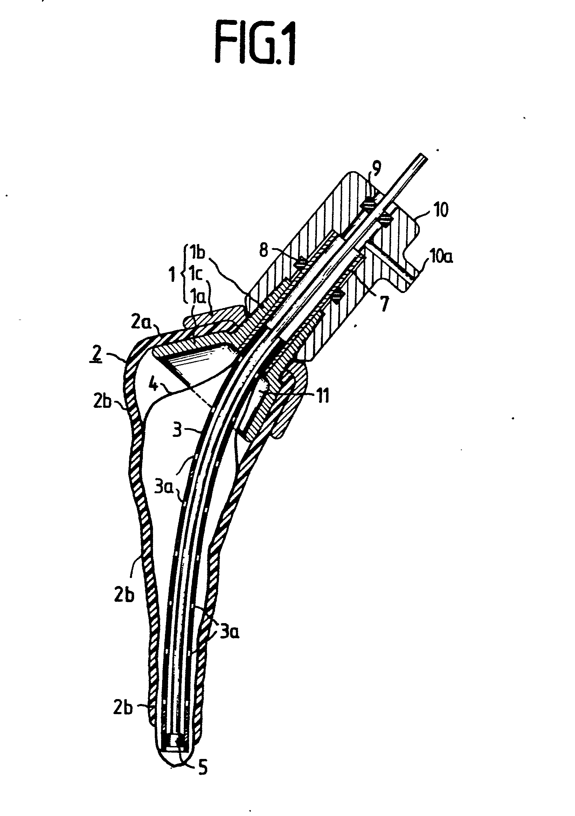

Nach Beendigung der in Figur 3 gezeigten Phase werden die nicht mehr benötigten Hilfsmittel, also die Teile 3 bis 10 entfernt, wofür bezüglich der Teile 3 bis 7 ein Kanal (15 in Figur 4) im Anpaßteil 1 vorgesehen ist. Er ist von dem Gewindehals 1b (Figur 1) umgeben, auf welchen die Montagehilfe 10 aufschraubbar ist für die Bewerkstelligung der Vorgänge des Anpressens des Prepreg-Teiles 2 und der Aushärtung. Nach dem Aushärten wird die Montagehilfe 10 abgeschraubt und nach dem Entfernen der restlichen Hilfsmittel stattdessen ein Gelenkkopf, z.B. eine Aluminiumoxid-Keramikkugel aufgeschraubt, wie es in Figur 4 zur Darstellung des Endzustandes gezeigt ist. Der Gewindehals 1b weist zu diesem Zweck ein selbsthemmendes, konisches Gewinde auf.

Der weitere Verlauf der Operation, einschließlich des Einsetzens der Hüftpfanne, geschieht in der herkömmlichen Art und Weise.

Während bei den bekannten Hüftgelenkprothesen die Krafteinleitung in den Knochen unter Ausbildung von Querkräften geschieht, denen der Knochen in der Regel nicht gewachsen ist, erfolgt die Krafteinleitung bei der Gelenkprothese nach der Erfindung in der physiologisch richtigen Richtung, wobei die Prothese einerseits ein großes Trägheitsmoment aufweist und andererseits die Einleitungskräfte pro Flächeneinheit wegen der großen beteiligten Flächen gering sind. Querbelastungen des Knochens und eine mehr oder weniger punktförmige Kraftübertragung wie bei herkömmlichen Prothesen werden vermieden, zumal die Struktur selbst elastisch ist und damit den elastischen Verformungen des Knochens folgen kann.

Eine Reparatur der erfindungsgemäßen Prothese ist im Gegensatz zu den bekannten Prothesen durch Ersatz einzelner Teile möglich. Darüber hinaus kann später eine neue Prothese wegen des zur Verfügung stehenden Hohlraumes ohne weiteres eingebracht werden, wobei diese herkömmlicher Art sein oder auch der erfindungsgemäßen Ausführungsform entsprechen kann.

* * * * *

Patentansprüche

1.    Gelenkprothese,

gekennzeichnet durch folgende Bestandteile:

a) wenigstens ein Anpaßteil (1),

b) wenigstens ein mit dem Anpaßteil (1) verbundenes und in
denjenigen Bereichen (2b) aushärtbares Prepreg-Teil (2),
die dem Knochenhohlraum (13) anzupassen sind, in den die
Gelenkprothese einzusetzen ist,

c) für ein Strömungsmittel weitgehend undurchlässige und
durch dieses aufblähbare Anpreßmittel (4) zum Anpressen
der noch unausgehärteten Bereiche (2b) des Prepreg-Teiles
(2) mittels des zuführ- und/oder absaugbaren Strömungsmittels.

2.    Gelenkprothese nach Anspruch 1, d a d u r c h   g e -
k e n n z e i c h n e t ,  daß wenigstens eine Einführhilfe (3), welche in die weitgehend für Strömungsmittel
undurchlässigen Anpreßmittel (4) einführbar oder in diese
eingeführt und steifer ausgestaltet ist als die Anpreßmittel (4).

3.    Gelenkprothese nach einem der vorangehenden Ansprüche, d a d u r c h   g e k e n n z e i c h n e t ,  daß

ME31.1-EU                    Seite 12

das Prepreg-Teil (2) mindestens in demjenigen Bereich (2a), in dem es vom Anpaßteil (1) gehalten ist, schlauch- oder ring-, insbesondere glockenförmig ist und/oder bereits vorausgehärtet ist.

4.    Gelenkprothese nach einem der vorangehenden Ansprüche, d a d u r c h   g e k e n n z e i c h n e t ,   daß das Prepreg-Teil (2) mindestens am noch unausgehärteten Ende streifenförmige und/oder in Form von wechselnd gegenläufigen Spiralen, die miteinander verwoben sind, ausgebildete Bereiche (2b) aufweist.

5.    Gelenkprothese nach einem der vorangehenden Ansprüche, d a d u r c h   g e k e n n z e i c h n e t ,   daß ein Einführungsteil (3) für Aushärtemittel (5) für die unausgehärteten Bereiche (2b) des Prepreg-Teiles (2) vorgesehen ist.

6.    Gelenkprothese nach Anspruch 5, d a d u r c h   g e k e n n z e i c h n e t ,   daß das Einführungsteil zugleich als Einführhilfe (3) und/oder Einleitungsteil für das zuzuführende Strömungsmittel ausgebildet ist.

7.    Gelenkprothese nach einem der vorangehenden Ansprüche, d a d u r c h   g e k e n n z e i c h n e t ,   daß das Aushärtemittel (5) als UV-Licht- und/oder Ultraschall-Strahler ausgebildet ist und zwischen Strahler und Pre-

/13

preg-Teil (2) befindliche Teile (3, 4) für die Strahlung durchlässig sind.

8. Gelenkprothese nach einem der Ansprüche 6 oder 7, d a d u r c h   g e k e n n z e i c h n e t , daß das Einführungsteil als Führungselement für den Strahler (5) ausgebildet ist.

9. Gelenkprothese nach einem der vorangehenden Ansprüche, d a d u r c h   g e k e n n z e i c h n e t , daß das Einführungsteil (3) derart hohl geformt ist, daß das Aushärtemittel (5) hindurchführbar ist.

10. Gelenkprothese nach einem der vorangehenden Ansprüche, d a d u r c h   g e k e n n z e i c h n e t , daß die für Strömungsmittel weitgehend undurchlässigen Anpreßmittel (4) im aufgeblähten, aber nicht merklich gedehnten Zustand erweitert ausgebildet sind im Vergleich zu dem bestimmungsgemäß zur Aufnahme von Gelenkprothesenteilen vorgesehenen Knochenhohlraum (13) und/oder weniger weit ausgebildet sind als der bestimmungsgemäß zur Aufnahme von Gelenkprothesenteilen vorgesehene Knochenhohlraum (13) und/oder beutelförmig ausgebildet sind.

11. Gelenkprothese nach einem der vorangehenden Ansprüche, d a d u r c h   g e k e n n z e i c h n e t , daß die weitgehend Strömungsmittel undurchlässigen Anpreßmittel (4) aus Folie bestehen.

12. Gelenkprothese nach einem der vorangehenden Ansprüche, d a d u r c h  g e k e n n z e i c h n e t , daß das Prepreg-Teil (2) zugleich als weitgehend für Strömungsmittel undurchlässiges Anpreßmittel (4) ausgebildet ist.

13. Gelenkprothese nach einem der vorangehenden Ansprüche, d a d u r c h  g e k e n n z e i c h n e t , daß das Anpaßteil (1) einen Kanal (15) für die Einführhilfe (3) ringförmig umgibt und/oder ein Tragelement für Endoprothesenteile (Gelenkkopf 16) bildet.

14. Gelenkprothese nach Anspruch 13, d a d u r c h  g e - k e n n z e i c h n e t , daß durch den Kanal (15) hindurch mindestens eines der folgenden Bauteile entfernbar ist: die Einführhilfe (3), das Einführungsteil (3), die Aushärtemittel (5), die Transportmittel (6), die Anpreßmittel (4).

15. Gelenkprothese nach einem der Ansprüche 13 oder 14, d a d u r c h  g e k e n n z e i c h n e t , daß der Kanal (15) innerhalb eines Stutzens (Gewindehals 1b) des Anpaßteiles (1) angeordnet ist, der lösbar mit einer Montagehilfe (10) verbindbar ist.

*  *  *  *  *

# FIG.1

FIG.4

FIG.3

FIG.2

**Europäisches Patentamt**

**EUROPÄISCHER RECHERCHENBERICHT**

**0061993**

Nummer der Anmeldung

EP 82 73 0043

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl. ³) |
|---|---|---|---|
| A | DE-A-2 305 441 (ROSENTHAL STEMAG TECHNISCHE KERAMIK) | 1 | A 61 F 1/03 |
| | --- | | |
| A | CH-A- 389 231 (DOERPINGHAUS) | 1 | |
| | ----- | | |

| RECHERCHIERTE SACHGEBIETE (Int. Cl. ³) |
|---|
| A 61 F |
| B 29 G |

Der vorliegende Recherchenbericht wurde für alle Patentansprüche erstellt.

| Recherchenort | Abschlußdatum der Recherche | Prüfer |
|---|---|---|
| DEN HAAG | 05-07-1982 | STEENBAKKER J. |

KATEGORIE DER GENANNTEN DOKUMENTEN

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1503. 03.82